# EUROPEAN PATENT SPECIFICATION

(11) **EP 2 311 454 B1**
(45) Date of publication and mention of the grant of the patent: **18.03.2015**
(21) Application number: 09765946.0
(22) Date of filing: 19.06.2009
(51) Int. Cl.: A61K 31/405, A61P 17/00

(54) **DERMATOLOGICAL PHARMACEUTICAL COMPOSITION FOR THE TREATMENT OF SKIN INFLAMMATION DISEASES, SUCH AS DERMATITIS, ATOPIC DERMATITIS, VITILIGO, ALOPECIA AREATA, ACNE, PSORIASIS, PRURITUS OR COMBINATIONS OF SAME**
DERMATOLOGISCHE PHARMAZEUTISCHE ZUSAMMENSETZUNG ZUR BEHANDLUNG VON ENTZÜNDLICHEN HAUTERKRANKUNGEN WIE DERMATITIS, ATOPISCHE DERMATITIS, VITILIGO, ALOPECIA AREATA, AKNE, PSORIASIS, PRURITUS ODER KOMBINATIONEN DAVON
COMPOSITION PHARMACEUTIQUE DERMATOLOGIQUE DESTINÉE AU TRAITEMENT DE PATHOLOGIES INFLAMMATOIRES CUTANÉES TELLES QUE LA DERMATITE, LA DERMATITE ATOPIQUE, LE VITILIGO, L'ALOPÉCIE AREATA, L'ACNÉ, LE PSORIASIS ET LE PRURIT, ET DE COMBINAISONS DE CES DERNIÈRES

(30) Priority: 20.06.2008 ES 200801861
(43) Date of publication of application: 20.04.2011
(73) Proprietor: Umbert Millet, Ignacio, 08017 Barcelona (ES)
(72) Inventor: Umbert Millet, Ignacio, 08017 Barcelona (ES)
(74) Representative: Curell Suñol S.L.P.
(86) International application number: PCT/ES2009/000339
(87) International publication number: WO 2009/153373

(56) References cited:
- EP-A1- 1 864 657
- WO-A1-87/02891
- WO-A1-2006/096360
- WO-A2-02/074290
- WO-A2-2008/030359
- JP-A- 2007 291 069
- US-A1- 2005 014 729
- DRAELOS ET AL: "Novel topical therapies in cosmetic dermatology", CURRENT PROBLEMS IN DERMATOLOGY, MOSBY, vol. 12, no. 5, 1 September 2000 (2000-09-01), - 29 December 2011 (2011-12-29), pages 235-239, XP005466383, ISSN: 1040-0486, DOI: 10.1016/S1040-0486(00)80007-1
- KEMENY, L. ET AL.: 'Pharmacological studies on dithranol-induced irritative dermatitis in mice' ARCH. DERMATOL. RES. vol. 281, 1989, pages 362 - 365, XP008141695
- BRALLEY EVE E ET AL: "Topical anti-inflammatory activity of Polygonum cuspidatum extract in the TPA model of mouse ear inflammation", JOURNAL OF INFLAMMATION, BIOMED CENTRAL, LONDON, GB, vol. 5, no. 1, 8 February 2008 (2008-02-08), page 1, XP021037122, ISSN: 1476-9255
- CHEN ANPING ET AL: "Activation of PPAR{gamma} by curcumin inhibits Moser cell growth and mediates suppression of gene expression of cyclin D1 and EGFR", AMERICAN JOURNAL OF PHYSIOLOGY: GASTROINTESTINAL AND LIVER PHYSIOLOGY, AMERICAN PHYSIOLOGICAL SOCIETY, US, vol. 288, no. 3, 1 March 2005 (2005-03-01) , pages G447-G456, XP002468080, ISSN: 0193-1857, DOI: 10.1152/AJPGI.00209.2004
- JACOB ASHA ET AL: "Mechanism of the Anti-inflammatory Effect of Curcumin: PPAR-gamma Activation", 1 January 2007 (2007-01-01), PPAR RESEARCH, HINDAWI PUBLISHING CORP, US, PAGE(S) 5PP, XP009100465, ISSN: 1687-4757
- KURODA MINPEI ET AL: "Hypoglycemic effects of turmeric (Curcuma longa L. rhizomes) on genetically diabetic KK-Ay mice.", May 2005 (2005-05), BIOLOGICAL & PHARMACEUTICAL BULLETIN MAY 2005, VOL. 28, NR. 5, PAGE(S) 937 - 939 ISSN: 0918-6158
- ZHENG SHIZHONG ET AL: "Activation of PPARgamma is required for curcumin to induce apoptosis and to inhibit the expression of extracellular matrix genes in hepatic stellate cells in vitro.", 15 November 2004 (2004-11-15), THE BIOCHEMICAL JOURNAL 15 NOV 2004, VOL. 384, NR. PT 1, PAGE(S) 149 - 157 ISSN: 1470-8728
- GE ET AL: "Resveratrol inhibits macrophage expression of EMMPRIN by activating PPARgamma", VASCULAR PHARMACOLOGY, ELSEVIER, AMSTERDAM, NL, vol. 46, no. 2, 20 December 2006 (2006-12-20), pages 114-121, XP005810114, ISSN: 1537-1891, DOI: 10.1016/J.VPH.2006.08.412
- PERSHADSINGH H A ET AL: "[alpha]-lipoic acid is a weak dual PPAR[alpha]/[gamma] agonist an ester derivative with increased PPAR[alpha]/[gamma] efficacy and antioxidant activity", 2005, JOURNAL OF APPLIED RESEARCH 2005 US, VOL. 5, NR. 4, PAGE(S) 510 - 523 ISSN: 1537-064X
- HSIEH ET AL: "Low and high levels of alpha-tocopherol exert opposite effects on IL-2 possibly through the modulation of PPAR-gamma, IkappaBalpha, and apoptotic pathway in activated splenocytes", NUTRITION, ELSEVIER INC, US, vol. 22, no. 4, 1 April 2006 (2006-04-01), pages 433-440, XP005331435, ISSN: 0899-9007, DOI: 10.1016/J.NUT.2005.10.001
- LI ET AL: "Green tea leaf extract improves lipid and glucose homeostasis in a fructose-fed insulin-resistant hamster model", JOURNAL OF ETHNOPHARMACOLOGY, ELSEVIER IRELAND LTD, IE, vol. 104, no. 1-2, 8 March 2006 (2006-03-08), pages 24-31, XP005290567, ISSN: 0378-8741, DOI: 10.1016/J.JEP.2005.08.045
- LI X ET AL: "Medicine for promoting synthesis of human self-body EPA and DHA", WPI / THOMSON,, vol. 2003, no. 21, 20 November 2002 (2002-11-20), XP002595954,
- MEZEI O ET AL: "Soy Isoflavones Exert Antidiabetic and Hypolipidemic ffects through the PPAR Pathways in Obese Zucker Rats andMurine RAW 264.7 Cells", THE JOURNAL OF NUTRITION, AMERICAN SOCIETY FOR NUTRITION, US, vol. 133, no. 5, 1 May 2003 (2003-05-01), pages 1238-1243, XP002317912, ISSN: 0022-3166
- MORIKAWA ET AL: "Growth arrest and apoptosis induced by quercetin is not linked to adipogenic conversion of human preadipocytes", METABOLISM, CLINICAL AND EXPERIMENTAL, W.B. SAUNDERS CO., PHILADELPHIA, PA, US, vol. 56, no. 12, 12 November 2007 (2007-11-12), pages 1656-1665, XP022331366, ISSN: 0026-0495, DOI: 10.1016/J.METABOL.2007.07.008
- SHEN P ET AL: "Differential Effects of Isoflavones, from Astragalus Membranaceus and Pueraria Thomsonii, on the Activation of PPARalpha, PPARgamma, and A Adipocyte Differentiation in Vitro", THE JOURNAL OF NUTRITION, AMERICAN SOCIETY FOR NUTRITION, US, vol. 136, no. 4, 1 December 2006 (2006-12-01), pages 899-905, XP008139243, ISSN: 0022-3166
- SHEN W ET AL: "R-[alpha]-Lipoic acid and acetyl-l-carnitine complementarily promote mitochondrial biogenesis in murine 3T3-L1 adipocytes", DIABETOLOGIA ; CLINICAL AND EXPERIMENTAL DIABETES AND METABOLISM, SPRINGER, BERLIN, DE, vol. 51, no. 1, 17 November 2007 (2007-11-17), pages 165-174, XP019558692, ISSN: 1432-0428, DOI: 10.1007/S00125-007-0852-4
- ULRICH SANDRA ET AL: "Peroxisome proliferator-activated receptor gamma as a molecular target of resveratrol-induced modulation of polyamine metabolism.", 15 July 2006 (2006-07-15), CANCER RESEARCH 15 JUL 2006, VOL. 66, NR. 14, PAGE(S) 7348 - 7354 ISSN: 0008-5472
- CAMPBELL SHARON E ET AL: "Gamma ([gamma]) tocopherol upregulates peroxisome proliferator activated receptor (PPAR) gamma ([gamma]) expression in SW 480 human colon cancer cell lines", BMC CANCER, BIOMED CENTRAL, LONDON, GB, vol. 3, no. 1, 1 October 2003 (2003-10-01) , page 25, XP021004590, ISSN: 1471-2407, DOI: 10.1186/1471-2407-3-25
- VENKATRAMAN MEENAKSHI S ET AL: "alpha-Lipoic acid-based PPARgamma agonists for treating inflammatory skin diseases", ARCHIVES OF DERMATOLOGICAL RESEARCH, SPRINGER, INTERNATIONAL, BERLIN, DE, vol. 296, no. 3, 1 August 2004 (2004-08-01), pages 97-104, XP002459339, ISSN: 0340-3696, DOI: 10.1007/S00403-004-0480-5

## Description

### Technical field of invention

The present invention relates to a pharmaceutical composition for treating dermatological inflammatory diseases of the skin, such as for example dermatitis, atopic dermatitis, vitiligo, alopecia areata, acne, psoriasis and pruritus, and combinations of them, which contain at least one anti-inflammatory base, such as Indometacin.

### Background of the Invention

In dermatology, today there are frequent multiple inflammatory diseases of neurogenic origin. These diseases can be dermatitis, atopic dermatitis, vitiligo, alopecia areata, acne, psoriasis and pruritus, etc., according to immunological alterations, gene activations, infections, destruction of melanocytes destruction of immunoprivilege of the hair follicle, etc. occur.

There are currently multiple embodiments of drugs for the treatment of each of these diseases, mainly based on the use of an anti-inflammatory, such as indometacin, ibuprofen (although this is very photosensitive), their equivalents, or corticoids, topically.

In dermatology it is not usual the administration of combinations of active ingredients. This is generally because of the difficulty to found by the person skilled in the art the combination of two or more active ingredients, with respect to the chemical stability and interactions that can cause drug products to be present in the same formulation (see FR2848454 of L'Oréal) .

There is therefore in dermatology the perception that the association of active ingredients is generally not effective against skin diseases.

Therefore, the formulations against inflammatory diseases in dermatology are based on anti-inflammatories, corticoids alone or combined (for example, with doxepin, for example in CN1363276) of powerful action, not without side effects.

WO 2006/096360 (A1) describes topical alcoholic gel compositions that are useful for delivering therapeutic levels of an NSAID to target in and below the skin. The compositions comprise a topically active drug, an alcoholic solvent, a polymeric thickener, and optionally a keratolytic agent. In one embodiment, excellent viscosity for dermal application is attained without the need of a step for neutralizing the pH of the composition. Alcoholic and alcohol-free topical compositions comprising an NSAID prodrug are also disclosed. The compositions are particularly useful for the treatment of pseudo folliculitis barbae.

WO 2008/030359 (A2) describes topical compositions that are useful for delivering a therapeutic level of an NSAID to a target within a subject having a local inflammatory disorder. The composition comprises a drug and a solvent system, wherein the solvent system comprises at least two solvent alcohols and wherein the solvent system is present in an amount sufficient to solubilize the drug, the solvent system is a low alkanol system, and the composition is a single phase composition. Exemplary solvent systems are those for which one of the at least two solvent alcohols is polyethylene glycol, glycerin, butylene glycol, diproylene glycol, propylene glycol, ethanol, isopropanol, or a derivative thereof. The local inflammatory disorder can be pseudofolliculitis barbae, dermatitis, psoriasis, wounds, or sunburn.

The present invention aims to disclose a pharmaceutical compound that overcomes this barrier, and is suitable for a very effective treatment of many diseases, concomitant or not, of inflammatory kind, mainly but not exclusively, arising from new known etiologies, for example allostatic overload by neuroimmunoendocrine stress.

### Summary of the invention

To this end, the object of the present invention is a new pharmaceutical composition for treating dermatological inflammatory diseases of the skin, such as for example dermatitis, atopic dermatitis, vitiligo, alopecia areata, acne, psoriasis and pruritus comprising an anti-inflammatory such as indometacin, characterized in that it further comprises
- a combination of topical antioxidants to boost the anti-inflammatory effect by the activation of the peroxisome proliferator-activated receptor gamma (PPAR-γ) ; and
- one or more optional active ingredients selected alternately from:
- at least one corticoid; and
- an antibiotic.

The compositions according to the invention also comprise preferably at least one antioxidant substance with keratinocyte anti-proliferative effect, and a blocking substance of tumor necrosis factor alpha (TNF-α) or other cytokines stimulant of inflammatory reactions, also with anti-proliferative effects.

The preferred antioxidant substance with keratinocytes anti-proliferative effect is manganese, particularly in the case of psoriasis, and the preferred blocking substance of tumor necrosis factor alpha (TNF-α), also with anti-proliferative effect is pentoxifylline. Manganese is part of enzymes such as superoxide dismutase SOD, which show a high antioxidant capacity and protection against free radicals, with anti-proliferative effect.

Manganese and pentoxifylline are included in weight proportions of up to 5%.

According to another feature of the present invention, at least one of the topical antioxidants is an activator of the peroxisome proliferator-activated receptor gamma (PPAR-γ), such as lipoic acid.

In a first variant of the drug, designed particularly for the treatment of dermatitis, atopic dermatitis, vitiligo, alopecia areata, psoriasis and pruritus, said optional active ingredients are corticoids.

In a second variant, corticoids are replaced by an antibiotic for the treatment of acne, as inflammatory process of the skin.

In case of corticoids, clobetasol propionate can be used, especially for the treatment of vitiligo, alopecia areata, and psoriasis, in a decreasing concentration with decrease of the pathological intensity, preferably between 0.1 and 0% by weight.

For the treatment of dermatitis and atopic dermatitis, corticoids are preferably a combination of hydrocortisone and triamcinolone acetonide, particularly in a decreasing concentration with decrease in the pathological intensity, between 0.5 and 0% by weight.

According to another feature of the present invention, in the first variant doxepin can be added, especially for the treatment of atopic dermatitis, especially between 0 and 5% by weight.

Topical antioxidants are selected from: quercetin, catechins from green tea, lipoic acid, curcumin, ascorbyl palmitate, Coenzyme Q10, resveratrol, pycnogenol^{®}, L- carnosine, taurine, vitamin E, vitamin C, papaya extract, isoflavones and lycopene. Manganese is also expected to be use as an antioxidant in the case of psoriasis.

The preferred combinations of topical antioxidants of formulations of the present invention are:
- 5% ascorbyl palmitate, 5% vitamin E, and 3% lycopene,
- 1% lycopene, 5% vitamin E, and 3% vitamin C,
- 1% resveratrol,
- 3-5% lipoic acid,
- Coenzyme Q10 3%, 5% vitamin E, and 5% taurine,
- C.s.p % papaya extract,
- 0.5% green tea catechins,
- 1% pycnogenol^{®}, 3% vitamin C, and 5% ascorbyl palmitate,
- 1% curcumin,
- 2% L- carnosine, 3% vitamin C, 5% vitamin. E, and 5% ascorbyl palmitate,
- 5%isoflavones, 0.5% green tea catechins; 5% ascorbyl palmitate, and
- 1% quercetin
that the medical practitioner will select depending on the intensity of the inflammatory disease.

The dermatological pharmaceutical composition of the present invention may further comprise
- an emollient selected from: glycerin, aloe vera, propylene glycol, and lactic acid
- an adjunctive of anti-inflammatory, such as omega-3,
- an antifungal such as ketoconazole, and
- L-carnitine.

In the case of the first variant, with corticoids, the pharmaceutical composition according to the invention can also include an antibiotic such as gentamicin, ciprofloxacin, clindamycin, or equivalents.

In the case of the second option, free of corticoids, the antibiotic is preferably selected from the group consisting of: ciprofloxacin, clindamycin, sodium sulfacetamide, gentamicin and erythromycin.

In both cases, the drug according to the invention can also contain nicotinamidae.

The new dermatological pharmaceutical compositions of the present invention are completely inventive, because until now, all would suggest a dermatologist skilled in the art that the association of these components would have an adverse or anti-synergistic effect.

The effects of a combination of antioxidants are a stimulation of the activation of PPAR-γ and an increase of the anti-inflammatory efficacy. Thus, generally the resulting formulations of the present invention can reduce the weight proportions of aggressive substances such as steroids, antibiotics and effective doxepin, while each of these ingredients acts on one or a few specific diseases, potentiating surprisingly, possibly by a synergistic effect, the anti-inflammatory effect and allowing the formulation in accordance with the intensity of each disease.

### Description of preferred embodiments

The object of the invention is a dermatological drug product useful for the treatment of inflammatory diseases of the skin, such as for example dermatitis, atopic dermatitis, vitiligo, alopecia areata, acne, psoriasis, pruritus, etc., comprising the following active ingredients,
- a base anti-inflammatory, such as indometacin,
- one or more antioxidants with anti-proliferative effect of keratinocytes,
- a TNF-α anti-proliferative and blocking substance (or other cytokines that produce inflammations)
- an active ingredient selected from:
- an antibiotic for the treatment of acne, as inflammatory process of the skin, and
- one or more corticoids for the rest of diseases, and
- a combination of topical antioxidants, enhancers of the activation of PPAR-γ.

Suitable emollients and excipients are also added.

The preferred anti-proliferative antioxidant is manganese, especially useful for psoriasis. It must be pointed out that manganese is part of enzymes such as superoxide dismutase SOD, which shows a high antioxidant capacity and protection against free radicals, with anti-proliferative effect. In this regard, substances that are part of SOD type enzymes with antioxidant capacity should be seen as technical equivalents of manganese.

The preferred TNF-α anti-proliferative and blocking substance is pentoxifylline, which shall not be applied in the case of acne.

The corticoid is high power, clobetasol propionate for the treatment of vitiligo, alopecia areata, and psoriasis, in a decreasing concentration with decrease of the pathological intensity. The proportions are between 0 and 0.1% by weight of clobetasol propionate.

The corticoid will be of low power, hydrocortisone, and/or medium power, triamcinolone acetonide for treating atopic dermatitis, in a decreasing concentration with decrease in the pathological intensity. It will contain between 0 and 0.5% by weight of triamcinolone acetonide, and between 0 and 2% by weight of hydrocortisone.

For the acute stages of the disease, the formulations include corticoids, although with a tendency to their rapid removal or reduction, and maintenance stages they are deleted from the formulation.

For the treatment of atopic dermatitis, the formula will include up to 5% by weight of doxepin, to block the inflammation, especially in the case of atopic dermatitis. Proportions above 5% could be toxic.

For the treatment of psoriasis varying proportions up to 5% by weight of manganese will be included, because of its important antioxidant and anti-proliferative combined action.

More than one of the topical antioxidants is an activator of PPAR-γ. A non-limitative example is lipoic acid, whose inclusion in a topical formulation is completely new.

Generally, and as it will be appreciated, a very important and innovative characteristic of the formulation of the invention is that it is formulated with a variety of active ingredients that block corresponding receptors for mast cells, to control the multiple inflammation with different simultaneous etiologies, or origin in neurological stress, and with different disease manifestations, possibly simultaneously.

For example, the lipoic acid regulates the peroxisome proliferator-activated receptor gamma (PPAR-γ).

As a regulator of sebaceous secretion proportions of nicotinamide can be included.

As excipient propylene glycol can also be added, which acts as a solvent to increase the solubility of the active ingredients.

### Examples

### Example 1: Family of anti-inflammation formulas (psoriasis, vitiligo, alopecia areata)

A) Common active ingredients (Formula Skeleton).
   - Corticoids:
      - Clobetasol propionate ... 0.05% (high power)
   - Indometacin ... 1-3% (anti-inflammatory)
   - Pentoxifylline ... 1-3% (anti-proliferative)
   - Antioxidants (combinations of):
      - Lipoic acid ... 3-5%
      - Quercetin ... 0.1 to 5%
      - Green Tea Catechins ... 0.5%
      - Curcumin ... 1%
      - Ascorbyl Palmitate ... 5%
      - Coenzyme Q10 ... 0.3%
      - Resveratrol ... 1%
      - Pycnogenol^{®} ... 1%
      - L- Carmosine ... 2%
      - Taurine ... 0.5%
      - Isoflavones ... 5%
      - Lycopene ... 1%
      - Papaya ... Q.s.
      - Other antioxidants
   - Excipients: Base Beeler^{®}, Orabase^{®}, water-alcohol solution
B) Additional Active Ingredients
   - Emollients:
      - Glycerin ... 5-15%
      - Aloe Vera ... 5-15%
      - Propilengicol ... 5-20%
      - Lactic Acid ... 5-12%
      - Omega3 ... 5-10%
   - Doxepin ... 1-5%
   - Ginseng Extract ... 1-2%
   - Ketoconazole (antifungal) ... 0.1-2%
   - Nicotinamide ... 2%
   - L-carnitine ... 1%
   - Gentamicin (antibiotic) ... 0.1%
   - Manganese (Psoriasis) 0.01 to 5%

### Example 2: Family of maintenance formulas (psoriasis, vitiligo, alopecia areata)

A) Common active ingredients (Formula Skeleton).
   - Indometacin ... 1-3% (anti-inflammatory)
   - Pentoxifylline ... 1-3% (anti-proliferative)
   - Antioxidants (combinations of):
      - Lipoic acid ... 3-5%
      - Quercetin ... 0.1 to 5%
      - Green Tea Catechins ... 0.5%
      - Curcumin ... 1%
      - Ascorbyl Palmitate ... 5%
      - Coenzyme Q10 ... 0.3%
      - Resveratrol ... 1%
      - Pycnogenol^{®} ... 1%
      - L- Carmosina ... 2%
      - Taurine ... 0.5%
      - Isoflavones ... 5%
      - Lycopene ... 1%
      - Other antioxidants
   - Excipients: Base Beeper^{®}, Orabase^{®}, water-alcohol solution
B) Supplementary active ingredients.
   - Emollients:
      - Glycerin ... 5-15%
      - Aloe Vera ... 5-15%
      - Propilengicol ... 5-20%
      - Lactic Acid ... 5-12%
      - Omega3 ... 5-10%
   - Doxepin ... 1-5%
   - Ginseng Extract ... 1-2%
   - Ketoconazole (antifungal) ... 0.1 -2%
   - Nicotinamide ... 2%
   - L-carnitine ... 1%
   - Gentamicin ... 0.1% (antibiotic)
   - Manganese (Psoriasis) ... 0.01 to 5%

### Example 3: Family of anti-inflammation formulas (atopic dermatitis or eczema)

A) Common active ingredients (Formula Skeleton).
   - Corticoids:
      - Triamcinolone acetonide 0.1% (medium power)
      - Hydrocortisone 1% (low power)
   - Indometacin ... 1-3% (anti-inflammatory)
   - Pentoxifylline ... 1-3% (anti-proliferative)
   - Antioxidants (combinations of):
      - Lipoic acid ... 3-5%
      - Quercetin ... 0.1 to 5%
      - Green Tea Catechins ... 0.5%
      - Curcumin ... 1%
      - Ascorbyl Palmitate ... 5%
      - Coenzyme Q10 ... 0.3%
      - Resveratrol ... 1%
      - Pycnogenol^{®} ... 1%
      - L-Carmosina ... 2%
      - Taurine ... 0.5%
      - Isoflavones ... 5%
      - Lycopene ... 1%
      - Vitamin E ... 1%
   - Excipients: Base Beeper^{®}
B) Additional Active Ingredients
   - Emollients:
      - Glycerine ... 5-15%
      - Aloe Vera ... 5-15%
      - Propilengicol ... 5-20%
      - Lactic Acid ... 5-12%
      - Omega3 ... 5-10%
   - Doxepin ... 1-5%
   - Ginseng Extract ... 1-2%
   - Ketoconazole (antifungal) ... 0.1-2%
   - Nicotinamide ... 2%
   - Gentamicin ... 0.1% (antibiotic)

### Example 4: Family of maintenance formulas (atopic dermatitis, or eczema)

A) Common active ingredients (Formula Skeleton).
   - Indometacin ... 1-3% (anti-inflammatory)
   - Pentoxifylline ... 1-3% (anti-proliferative)
   - Antioxidants (combinations of) :
      - Lipoic acid ... 3-5%
      - Quercetin ... 0.1 to 5%
      - Green Tea Catechins ... 0.5%
      - Curcumin ... 1%
      - Ascorbyl Palmitate ... 5%
      - Coenzyme Q10 ... 0.3%
      - Resveratrol ... 1%
      - Pycnogenol^{®} ... 1%
      - L- Carmosina ... 2%
      - Taurine ... 0.5%
      - Isoflavones ... 5%
      - Lycopene ... 1%
      - Vitamin E ... 1%
      - Other antioxidants
   - Excipients: Base Beeper^{®}.
B) Additional Active Ingredients
   - Emollients:
      - Glycerin ... 5-15%
      - Aloe Vera ... 5-15%
      - Propilengicol.... 5-20%
      - Lactic Acid ... 5-12%
      - Omega3 ... 5-10%
   - Doxepin ... 1-5%
   - Ginseng Extract ... z
   - Ketoconazole (antifungal) ... 0.1-2%
   - Nicotinamide ... 2%
   - Gentamicin ... 0.1% (antibiotic)

### Example 5: Family of formulas for treatment of acne

A) Common active ingredients (Formula Skeleton)
   - Antibiotic (only one) :
      - Ciprofloxacin ... 1%
      - Clindamycin ... 2%
      - Sodium Sulfacetamide ... 10%
      - Gentamicin ... 0.1%
      - Erythromycin ... 2%
   - Nicotinamide (Vit. PP) ... 4%
   - Antioxidants (combinations of):
      - Lipoic acid ... 3-5%
      - Quercetin ... 0.1 to 5%
      - Green Tea Catechins ... 0.5%
      - Curcumin ... 1%
      - Ascorbyl Palmitate ... 5%
      - Coenzyme Q10 ... 0.3%
      - Resveratrol ... 1%
      - Pycnogenol^{®} ... 1%
      - L- Carmosina ... 2%
      - Taurine ... 0.5%
      - Isoflavones ... 5%
      - Lycopene ... 1%
      - Vitamin E ... 1%
      - Other antioxidants
   - Excipient: Hydroalcoholic solution
B) Additional active ingredients
   - Anti-inflammatory: Indometacin
   - Propilengicol

In each case and for each patient, the doctor will assess - with appropriate regularity - the eventual presence and, where appropriate, the intensity of each of these diseases, and he/she will formulate the composition according to one or the other. For example, if the preeminent disease is psoriasis it will tend to include a greater proportion of manganese, with a higher proportion of ciobetasol propionate with a higher pathological intensity. If the disease is severe atopic dermatitis, it is preferable to formulate with doxepin and acetonide triamcinolone.

As the diagnosis improves, corticoids will be withdrawn gradually, in cases defined in Examples 1 and 3.

Also, the doctor will formulate the combination of topical antioxidants as a function of the intensity of the disease or diseases, which is valued with suitable frequency. Preferred combinations (wt%) of topical antioxidants are:
Low-power combinations for low-intensity inflammations:
   Combination 1: 5% ascorbyl palmitate, 5% vitamin E, 3% lycopene.
   Combination 2: 1% lycopene, 5% vitamin E, 3% vitamin C.

Medium-power combinations for medium intensity inflammations:
Combination 3: 1% resveratrol
Combination 4: 3-5% lipoic acid
Combination 5: 3% Coenzyme Q10, 5% vitamin E, 5% taurine
Combination 6: q.s.% papaya extract

High-power combinations for intensive inflammations:
Combination 7: 0.5% Green Tea Catechins
Combination 8: 1% pycnogenol^{®}, 3% vitamin C, 5% Ascorbyl Palmitate
Combination 9: 1% carcumin
Combination 10: 2% L-carnosine, 3% vitamin C, 5% vitamin E, 5% Ascorbyl Palmitate
Combination 11: 5% isoflavones, 0.5% green tea catechins, 5% ascorbyl palmitate
Combination 12: 1% quercetin.

Other combinations of other antioxidants are also possible.

Therefore, the present invention does not seeks the synergistic effect of the association of two active ingredients to treat a specific disease (e.g topical doxepin and corticoids, as described by Berberian and others), but a multiple synergistic effect on any disease in possible conjunction with other ones arising from new known etiologies, for example allostatic overload by neuroimmunoendocrine stress. Particularly, the topical antioxidants of the combination collaborate synergistically to cause the activation of PPAR-gamma and a consequent improvement of the anti-inflammatory effect. The person skilled in the art will understand that by the reformulation of the formula (% of corticoids and topical antioxidants), the practitioner can gain control of the inflammation, which was not possible in the current state of the art.

With the formulations of the present invention recurrence is reduced, the inflammation, proliferation and infection are controlled, without abuse of toxic substances with side effects, antibiotics, corticoids or doxepin in excess, as its effect is enhanced, surprisingly, on low proportions.

In the case of psoriasis and acne, an antibiotic is always included, because bacteria play an important role in these diseases.

## Claims

1. Dermatological pharmaceutical composition for use in the treatment of inflammatory diseases of the skin comprising indometacin as an anti-inflammatory, wherein said dermatological pharmaceutical composition further comprises:
- a combination of topical antioxidants to boost the anti-inflammatory effect,
- one or more optional active ingredients selected from: at least one corticoid or an antibiotic,
wherein at least one of the topical antioxidants is an activator of peroxisome proliferator-activated receptor gamma (PPAR-γ).

2. Dermatological pharmaceutical composition for use according to claim 1, wherein said one or more optional active ingredients are at least one corticoid for use in the treatment of inflammatory diseases of the askin selected from dermatitis, atopic dermatitis, vitiligo, alopecia areata, psoriasis and pruritus.

3. Dermatological pharmaceutical composition for use according to claim 2, wherein said at least one corticoid is clobetasol propionate for use in the treatment of inflammatory diseases of the skin selected from vitiligo, alopecia areata and psoriasis.

4. Dermatological pharmaceutical composition for use according to claim 2, wherein said at least one corticoid is a combination of hydrocortisone and triamcinolone acetonide for use in the treatment of inflammatory diseases of the skin selected from dermatitis and atopic dermatitis.

5. Dermatological pharmaceutical composition for use according to claim 2, further comprising doxepin for use in the treatment of atopic dermatitis.

6. Dermatological pharmaceutical composition for use according to claim 1, wherein said one or more optional active ingredients is an antibiotic for use in the treatment of acne.

7. Dermatological pharmaceutical composition
according to claim 1, wherein said antibiotic is selected from the group consisting of: ciprofloxacin, clindamycin, sulfacetamide sodium, gentamicin and erythromycin.

8. Dermatological pharmaceutical composition
according to any of the preceding claims, wherein said at least one topical antioxidant is selected from: greet tea catechins, lipoic acid, curcumin, ascorbyl palmitate, Coenzyme Q10, resveratrol, pycnogenol®, L-carnosine, taurine, vitamin E, vitamin C, papaya extract, isoflavones, manganese, lycopene, and quercetin.

9. Dermatological pharmaceutical composition,
according to claim 8, wherein said at least one topical antioxidant is selected from the group consisting of:
- 5% ascorbyl palmitate, 5% vitamin E, and 3% lycopene,
- 1% lycopene, 5% vitamin E, and 3% vitamin C,
- 1% resveratrol,
- 3-5% lipoic acid,
- 3% Coenzyme Q10, 5% vitamin E, and 5% taurine
- Q.s. % extract of papaya,
- 0.5% green tea catechins,
- 1% pycnogenol®; 3% vitamin C; and 5% ascorbyl palmitate,
- 1% curcumin
- 2% L- carnosine, 3% vitamin C, 5% vitamin. E and 5% ascorbyl palmitate,
- 5% isoflavones, 0.5% green tea catechins; 5% ascorbyl palmitate, and
- 1% quercetin

10. Dermatological pharmaceutical composition
according to any of the preceding claims, wherein said dermatological pharmaceutical composition further comprises said one or more of other antioxidant substance according to claim 1 with keratinocyte anti-proliferative effects, and at least one substance blocking the tumor necrosis factor alpha (TNF-α) or other cytokines which trigger the acute phase of the inflammatory reaction, also with anti-proliferative effect.

11. Dermatological pharmaceutical composition
according to claim 10, wherein said antioxidant substance with anti-proliferative effect is manganese in a proportion by weight of up to 5%, for use in treating psoriasis.

12. Dermatological pharmaceutical composition
according to claim 10, wherein said at least one substance blocking TNF-α is pentoxifylline in a proportion by weight of up to 5%.

13. Dermatological pharmaceutical composition
according to any of the previous claims, further comprising an emollient selected from glycerin, aloe vera, propylene glycol, and lactic acid and/or an omega-3 acid and/or an antifungal and/or L-carnitine and/or nicotinamide and/or an additional antibiotic selected from gentamicin, ciprofloxacin and clindamycin.

## Patentansprüche

1. Dermatologische pharmazeutische Zusammensetzung, die Indometacin als Antiphlogistikum umfasst, zur Verwendung bei der Behandlung entzündlicher Hautkrankheiten, wobei die dermatologische pharmazeutische Zusammensetzung des Weiteren umfasst:
- eine Kombination aus topischen Antioxidanzien zur Verstärkung der entzündungshemmenden Wirkung,
- mindestens einen optionalen Wirkstoff, ausgewählt aus: mindestens einem Corticoid oder einem Antibiotikum,
wobei mindestens eine der topischen Antioxidanzien ein Aktivator für den Peroxisomen-Proliferator-aktivierten Rezeptor Gamma (PPAR-γ) ist.

2. Dermatologische pharmazeutische Zusammensetzung zur Verwendung gemäß Anspruch 1, wobei der mindestens eine optionale Wirkstoff mindestens ein Corticoid zur Verwendung bei der Behandlung von entzündlichen Hautkrankheiten, ausgewählt aus Dermatitis, atopischer Dermatitis, Vitiligo, Alopecia Areata, Psoriasis und Pruritus, ist.

3. Dermatologische pharmazeutische Zusammensetzung zur Verwendung gemäß Anspruch 2, wobei das mindestens eine Corticoid Clobetasolpropionat zur Verwendung bei der Behandlung entzündlicher Hautkrankheiten, ausgewählt aus Vitiligo, Alopecia Areata und Psoriasis, ist.

4. Dermatologische pharmazeutische Zusammensetzung zur Verwendung gemäß Anspruch 2, wobei das mindestens eine Corticoid eine Kombination aus Hydrocortison und Triamcinolonacetonid zur Verwendung bei der Behandlung entzündlicher Hautkrankheiten, ausgewählt aus Dermatitis und atopischer Dermatitis, ist.

5. Dermatologische pharmazeutische Zusammensetzung zur Verwendung gemäß Anspruch 2, die weiterhin Doxepin zur Verwendung bei der Behandlung atopischer Dermatitis umfasst.

6. Dermatologische pharmazeutische Zusammensetzung zur Verwendung gemäß Anspruch 1, wobei der mindestens eine optionale Wirkstoff ein Antibiotikum zur Verwendung bei der Behandlung von Akne ist.

7. Dermatologische pharmazeutische Zusammensetzung zur Verwendung gemäß Anspruch 1, wobei das Antibiotikum ausgewählt ist aus der Gruppe bestehend aus: Ciprofloxacin, Clindamycin, Sulfacetamid-Natrium, Gentamicin und Erythromycin.

8. Dermatologische pharmazeutische Zusammensetzung zur Verwendung gemäß einem der vorangehenden Ansprüche, wobei die mindestens eine topische Antioxidanzie ausgewählt ist aus: Green Tea-Cathechinen, Liponsäure, Curcumin, Palmitinsäureascorbylester, Coenzym Q10, Resveratrol, Pycnogenol®, L-Carnosin, Taurin, Vitamin E, Vitamin C, Papaya-Extrakt, Isoflavonen, Mangan, Lycopin und Quercetin.

9. Dermatologische pharmazeutische Zusammensetzung zur Verwendung gemäß Anspruch 8, wobei die mindestens eine topische Antioxidanzie ausgewählt ist aus der Gruppe bestehend aus:
- 5 % Palmitinsäureascorbylester, 5 % Vitamin E und 3 % Lycopin,
- 1 % Lycopin, 5 % Vitamin E und 3 % Vitamin C,
- 1 % Resveratrol,
- 3-5 % Liponsäure,
- 3 % Coenzym Q10, 5 % Vitamin E und 5 % Taurin,
- Qs. % Papaya-Extrakt,
- 0,5 % Green Tea-Catechine,
- 1% Pycnogenol®; 3 % Vitamin C und 5 % Palmitinsäureascorbylester,
- 1% Curcumin,
- 2 % L-Carnosin; 3 % Vitamin C, 5 % Vitamin E und 5% Palmitinsäureascorbylester,
- 5 % Isoflavone, 0,5 % Green Tea-Catechine ; 5 % Palmitinsäureascorbylester und
- 1% Quercetin

10. Dermatologische pharmazeutische Zusammensetzung zur Verwendung gemäß einem der vorangehenden Ansprüche, wobei die dermatologische pharmazeutische Zusammensetzung weiterhin mindestens eine andere Antioxidanziensubstanz gemäß Anspruch 1 mit antiproliferativer Wirkung bei Keratinozyten umfasst und mindestens eine Substanz, die den Tumornekrosefaktor Alpha (TNF-α) oder andere Zytokine, welche die akute Phase einer Entzündungsreaktion auslösen, ebenfalls mit antiproliferativer Wirkung, blockiert.

11. Dermatologisch pharmazeutische Zusammensetzung zur Verwendung gemäß Anspruch 10, wobei die Antoixidanziensubstanz mit antiproliferativer Wirkung Mangan ist in einem Gewichtsverhältnis von bis zu 5 % zur Verwendung bei der Behandlung von Psoriasis.

12. Dermatologisch pharmazeutische Zusammensetzung zur Verwendung gemäß Anspruch 10, wobei die mindestens eine TNF-α blockierende Substanz Pentoxifyllin in einem Gewichtsverhältnis von bis zu 5 % ist.

13. Dermatologisch pharmazeutische Zusammensetzung zur Verwendung gemäß einem der vorangehenden Ansprüche, die weiterhin ein Linderungsmittel umfasst, ausgewählt aus Glycerin, Aloe Vera, Propylenglykol und Milchsäure und/oder eine Omega-3-Säure und/oder ein Antimykotikum und/oder L-Carnitin und/oder Nicotinamid und/oder ein zusätzliches Antibiotikum, ausgewählt aus Gentamicin, Ciprofloxacin und Clindamycin.

## Revendications

1. Composition pharmaceutique dermatologique pour son utilisation dans le traitement de maladies inflammatoires de la peau comprenant de l'indométacine en tant qu'anti-inflammatoire, dans laquelle ladite composition pharmaceutique dermatologique comprend en outre :
- une combinaison d'antioxydants topiques pour stimuler l'effet anti-inflammatoire,
- un ou plusieurs ingrédients actifs facultatifs sélectionnés parmi : au moins un corticoïde ou un antibiotique,
dans laquelle au moins un des antioxydants topiques est un activateur du récepteur gamma activé par les proliférateurs des peroxysomes (PPAR-γ).

2. Composition pharmaceutique dermatologique pour son utilisation selon la revendication 1, dans laquelle lesdits un ou plusieurs ingrédients actifs facultatifs sont au moins un corticoïde pour une utilisation dans le traitement de maladies inflammatoires de la peau sélectionnées parmi la dermatite, la dermatite atopique, le vitiligo, l'alopécie areata, le psoriasis et le prurit.

3. Composition pharmaceutique dermatologique pour son utilisation selon la revendication 2, dans laquelle ledit au moins un corticoïde est le propionate de clobétasol pour une utilisation dans le traitement de maladies inflammatoires de la peau sélectionnées parmi le vitiligo, l'alopécie areata et le psoriasis.

4. Composition pharmaceutique dermatologique pour son utilisation selon la revendication 2, dans laquelle ledit au moins un corticoïde est une combinaison d'hydrocortisone et d'acétonide de triamcinolone pour une utilisation dans le traitement de maladies inflammatoires de la peau sélectionnées parmi la dermatite et la dermatite atopique.

5. Composition pharmaceutique dermatologique pour son utilisation selon la revendication 2, comprenant en outre de la doxépine pour une utilisation dans le traitement de la dermatite atopique.

6. Composition pharmaceutique dermatologique pour son utilisation selon la revendication 1, dans laquelle lesdits un ou plusieurs ingrédients actifs facultatifs est/sont un antibiotique pour une utilisation dans le traitement de l'acné.

7. Composition pharmaceutique dermatologique pour son utilisation selon la revendication 1, dans laquelle ledit antibiotique est sélectionné dans le groupe consistant en : la ciprofloxacine, la clindamycine, le sulfacétamide sodique, la gentamicine et l'érythromycine.

8. Composition pharmaceutique dermatologique pour son utilisation selon l'une quelconque des revendications précédentes, dans laquelle ledit au moins un antioxydant topique est sélectionné parmi : des catéchines du thé vert, l'acide lipoïque, la curcumine, le palmitate d'ascorbyle, la coenzyme Q10, le resvératrol, le pycnogenol®, la L-carnosine, la taurine, la vitamine E, la vitamine C, l'extrait de papaye, des isoflavones, le manganèse, le lycopène et la quercétine.

9. Composition pharmaceutique dermatologique pour son utilisation selon la revendication 8, dans laquelle ledit au moins un antioxydant topique est sélectionné dans le groupe consistant en :
- palmitate d'ascorbyle à 5 %, vitamine E à 5 % et lycopène à 3 %,
- lycopène à 1 %, vitamine E à 5 % et vitamine C à 3 %,
- resvératrol à 1 %,
- acide lipoïque à 3-5 %,
- coenzyme Q10 à 3 %, vitamine E à 5 % et taurine à 5 %,
- extrait de papaye q.s.p. %,
- catéchines du thé vert à 0,5 %,
- pycnogenol® à 1 %, vitamine C à 3 % et palmitate d'ascorbyle à 5 %,
- curcumine à 1 %,
- L-carnosine à 2 %, vitamine C à 3 %, vitamine E à 5 % et palmitate d'ascorbyle à 5 %,
- isoflavones à 5 %, catéchines du thé vert à 0,5 %, palmitate d'ascorbyle à 5 %, et
- quercétine à 1 %.

10. Composition pharmaceutique dermatologique pour son utilisation selon l'une quelconque des revendications précédentes, dans laquelle ladite composition pharmaceutique dermatologique comprend en outre ladite une ou plusieurs autres substances antioxydantes selon la revendication 1 ayant des effets antiprolifératifs sur les kératinocytes, et au moins une substance bloquant le facteur de nécrose tumorale alpha (TNF-α) ou d'autres cytokines qui déclenchent la phase aiguë de la réaction inflammatoire, ayant également un effet antiprolifératif.

11. Composition pharmaceutique dermatologique pour son utilisation selon la revendication 10, dans laquelle ladite substance antioxydante ayant un effet antiprolifératif est le manganèse dans une proportion en poids allant jusqu'à 5 %, pour une utilisation dans le traitement du psoriasis.

12. Composition pharmaceutique dermatologique pour son utilisation selon la revendication 10, dans laquelle ladite au moins une substance bloquant le TNF-α est la pentoxifylline dans une proportion en poids allant jusqu'à 5 %.

13. Composition pharmaceutique dermatologique pour son utilisation selon l'une quelconque des revendications précédentes, comprenant en outre un émollient sélectionné parmi la glycérine, l'aloe vera, le propylène glycol et l'acide lactique et/ou un acide oméga-3 et/ou un antifongique et/ou la L-carnitine et/ou le nicotinamide et/ou un antibiotique supplémentaire sélectionné parmi la gentamicine, la ciprofloxacine et la clindamycine.
